Europäisches Patentamt

European Patent Office

Office européen des brevets

(11) Veröffentlichungsnummer: **0 121 788**
A2

# (12) EUROPÄISCHE PATENTANMELDUNG

(21) Anmeldenummer: 84102552.1

(22) Anmeldetag: 09.03.84

(51) Int. Cl.³: **C 07 G  7/00,** C 07 C  103/52, C 12 P  21/02

(30) Priorität: 14.03.83  DE 3309059

(43) Veröffentlichungstag der Anmeldung: **17.10.84** **Patentblatt 84/42**

(84) Benannte Vertragsstaaten: **AT BE CH DE FR GB IT LI NL SE**

(71) Anmelder: **HOECHST AKTIENGESELLSCHAFT, Postfach 80 03 20, D-6230 Frankfurt am Main 80 (DE)**

(72) Erfinder: **Vértesy, László, Dr., Eppenhainer Weg 6, D-6239 Eppstein/Taunus (DE)**
Erfinder: **Zepf, Karlheinz, Dr., Altkönigstrasse 3, D-6233 Kelkheim (Taunus) (DE)**

(54) **Verfahren zur Herstellung des Alpha-Amylaseinaktivators Tendamistat in kristalliner Form.**

(57) Verfahren zur Herstellung von kristallinem Tendamistat aus dem Kulturfiltrat von Kulturlösungen Tendamistat-produzierender Mikroorganismen ohne Chromatographie werden beschrieben. Das kristalline Tendamistat besitzt $\alpha$-Amylasehemmende Aktivität.

EP 0 121 788 A2

HOECHST AKTIENGESELLSCHAFT   HOE 83/F 036        Dr.D/Pa

Verfahren zur Herstellung des α-Amylaseinaktivators Tendamistat in kristalliner Form

In der deutschen Patentschrift 27 01890 sowie in den deutschen Offenlegungsschriften 30 38 130 und 31 07 106 werden ein peptidischer α-Amylaseinaktivator, der auch als HOE 467 und Tendamistat bezeichnet wird, und Verfahren zu seiner Herstellung beschrieben. In der Offenlegungsschrift 30 38 130 werden ferner die neuen, hochaktiven Komponenten HOE 467 A und HOE 467 B (Abbauprodukt von HOE 467 A) beschrieben. Sowohl HOE 467 als auch die Einzelkomponenten besitzen die Fähigkeit, Amylase wirksam zu hemmen. Aufgrund dieser Eigenschaften können die Verbindungen z.B. zur Regulierung des Blutzuckerspiegels verwendet werden.

In den folgenden Ausführungen steht HOE 467 bzw. Tendamistat sowohl für die Einzelkomponente als auch für das Gemisch.

Tendamistat ist ein Polypeptid. Die Isolierung und Reinigung von solchen Eiweiss-Stoffen ist in der Regel aufwendig, man ist bei der Gewinnung im allgemeinen auf chromatographische oder andere aufwendige Verfahren angewiesen (siehe Albert L. Lehninger: "Biochemie" Verlag Chemie, Weinheim, 1975, Seite 115 f). So sind auch in der deutschen Patentschrift 27 01 890 und der deutschen Offenlegungsschrift 30 38 130 chromatographische Reinigungs-Methoden an Ionenaustauschern bzw. Adsorptionsharzen beschrieben worden. Chromatographische Verfahren haben jedoch den Nachteil, daß sie in technischem Maßstab wegen ihrer Aufwendigkeit hohe Kosten verursachen, Energie-intensiv sind und letztendlich die Umwelt belasten.

Es wurde nun überraschenderweise gefunden, daß aus dem Kulturfiltrat von Kulturlösungen Tendamistat-produzierender Mikroorganismen nach Konzentrierung und Entsalzung

- 2 -

ohne anschließende chromatographische Verfahren Tendamistat direkt zur Kristallisation gebracht und rein gewonnen werden kann.

Die Erfindung betrifft daher ein Verfahren zur Herstellung des ⍺-Amylaseinaktivators Tendamistat in kristalliner Form sowie den nach diesem Verfahren hergestellten Inaktivator.

Besonders bevorzugt ist ein kristallines Tendamistat, das zum überwiegenden Teil, insbesondere zu 80 % oder mehr, aus HOE 467 A besteht.

Das Verfahren ist dadurch gekennzeichnet, daß man das Kulturfiltrat von Kulturlösungen Tendamistat-produzierender Mikroorganismen konzentriert und entsalzt und anschließend entweder

a) mit O bis 30 % eines geeigneten, mit Wasser mischbaren organischen Lösungsmittel versetzt, den pH-Wert dieser Lösung durch Zugabe einer in der Peptidchemie gebräuchlichen Säure auf 3,2 bis 5,5 erniedrigt oder auf 2 bis 3 erniedrigt und mit einer Base auf 3,2 bis 5,5 erhöht und das in der Lösung vorhandene Tendamistat, gegebenenfalls nach Zusatz von Impfkristallen, durch Stehenlassen zur Kristallisation bringt und abtrennt oder

b) mit einer in der Peptidchemie gebräuchlichen Säure auf einen pH-Wert zwischen 1,0 bis 3,0 einstellt, gegebenenfalls unlösliche Verunreinigungen abtrennt, die erhaltene Lösung mit einem für die Kristallisation von Eiweißstoffen üblichen Salz versetzt und das in der Lösung vorhandene Tendamistat, gegebenenfalls nach Zusatz von Impfkristallen, durch Stehenlassen zur Kristallisation bringt und abtrennt.

Das auskristallisierte Tendamistat wird z.B. durch Filtration oder Zentrifugation von der Mutterlauge getrennt und anschließend getrocknet. Der Wirkstoff ist -gewonnen durch dieses Verfahren- sehr rein. Falls erforderlich, kann er umkristallisiert und so nochmals gereinigt werden.

- 3 -

Durch Steuerung der Fermentationsparameter, z.B. der Fermentationszeit, kann im Kulturfiltrat der jeweilige Anteil an Komponenten HOE 467 A und HOE 467 B beeinflußt werden, so daß das Kulturfiltrat eine der Komponenten zu überwiegenden Teilen enthält.

Tendamistat-produzierende Mikroorganismen sind beispielsweise Streptomyces tendae ATCC 31 210 und Streptomyces tendae DSM 1912. Bei der Durchführung des erfindungsgemäßen Verfahrens werden insbesondere Kulturfiltrate der Kulturlösungen dieser beiden Stämme eingesetzt. Da sich der Wirkstoff hauptsächlich in der flüssigen, wäßrigen Phase der Kulturlösung befindet, wird der Feststoffanteil der Kulturlösung in bekannter Weise z.B. durch Abnutschen abgetrennt. Verfahren zur Herstellung der Kulturfiltrate werden beispielsweise in der deutschen Patentschrift 27 01 890 und Offenlegungsschrift 30 38 130 beschrieben. Die klare wäßrige Phase, das Kulturfiltrat, wird z.B. durch Destillation konzentriert und durch Dialyse entsalzt. Vorteilhaft ist auch das Ultrafiltrieren, wobei Konzentrieren und Entsalzen in einem Schritt durchgeführt werden können.

Das Verfahren a) wird zweckmäßig wie folgt durchgeführt: Das weitgehend entsalzte Kulturfiltrat erhalten nach der in der Patentschrift 27 01 390 oder Offenlegungsschrift 30 38 130 beschriebenen Methode und mit einem pH-Wert von 6-8 wird auf einen Gehalt von 3 bis 300 g Tendamistat pro Liter, vorzugsweise 20 bis 100 g/l Flüssigkeit konzentriert. 0 bis 30 %, vorzugsweise 5 - 15 %, eines geeigneten mit Wasser mischbaren organischen Lösungsmittels werden hinzugefügt. Von gegebenenfalls ausgefallenen Verunreinigungen wird abfiltriert und die Lösung wird gegebenenfalls anschließend nochmals konzentriert. Geeignete Lösungmittel sind z.B. niedermolekulare Alkohole wie Methanol, Ethanol, n-Propanol und Isopropanol und Ketone wie z.B. Aceton und Methylethylketon, besonders geeignet sind Isopropanol, Ethanol und Aceton. Der pH-Wert der Lösung wird auf 3,2 bis 5,5, vorzugsweise 4,2 bis 5,2, erniedrigt. Vorteilhaft ist so zu verfahren, daß die Säurezugabe nur bis zu dem pH-Wert erfolgt, bei welchem die Lösung noch eben klar bleibt (pH 4,6 bis 5,2).

- 4 -

0121788

Zur Einstellung des pH-Wertes eignen sich in der Peptidchemie gebräuchliche Säuren wie z.B. Schwefelsäure, Salzsäure und Essigsäure.

Stehenlassen der Lösung bei Temperaturen von $0^{o}C$ bis $60^{o}C$ vorzugsweise bei $5^{o}$ bis $25^{o}C$ führt zur Kristallisation. Es kann auch das Hinzufügen von (Impfkristallen) zum Ansatz von Vorteil sein. Nach 1 bis 50 Stunden ist die Kristallisation erfolgt. Sie kann gegegebenenfalls durch Korrektur des pH-Wertes auf 3,8 bis 5 sowie durch Abkühlen auf 0 bis $6^{o}C$ und erneutes Stehenlassen während 0,1 bis 14 Tagen vervollständigt werden. Die entstandene Kristallmasse wird dann in an sich bekannter Weise abgetrennt, gegebenenfalls umkristallisiert und getrocknet. Die Ausbeute beträgt je nach Verunreinigung des Ausgangsmaterials (Kulturfiltrats) bis über 95 % (bezogen auf den Gehalt im Kulturfiltrat).

Das geschilderte Verfahren ist eine Kristallisation der isoelektrischen Form des Tendamistats, dessen isoelektrischer Punkt für die Komponente HOE 467 A zu 4,35 bestimmt worden ist. Das Maximum der Schwerlöslichkeit dieser isoelektrischen Form ist naturgemäß bei pH 4,35.

Falls das entsalzte und konzentrierte Kulturfiltrat sehr verunreinigt ist, ist es vorteilhaft, die wäßrige oder wäßrige alkoholische bzw. wäßrige acetonische Lösung auf pH 2 bis 3 einzustellen. Das Tendamistat befindet sich in diesem pH-Bereich in Abwesenheit von Salzen klar in Lösung. Nun kann man durch Zugabe einer Lauge, wie z.B. Natronlauge den pH-Wert soweit anheben, daß wiederum die isoelektrische Kristallisation vonstatten gehen kann, also in den pH-Bereich 3,2 - 5,5, vorzugsweise 3,2 - 4,5.

Entsprechend Verfahren b) wird das entsalzte und konzentrierte Kulturfiltrat (erhalten z.B. nach der in der Patentschrift 27 01 390 bzw. Offenlegungsschrift 30 38 130 beschriebenen Methode), das das Tendamistat in wäßriger Lösung enthält, mit einer in der Peptidchemie gebräuchlichen Säure auf pH 1,0 bis 3,0, vorzugsweise auf pH 2,0 bis 2,8 eingestellt. Als Säuren eignen sich die für Verfahren a) genannten. Hierbei befindet sich

- 5 -

der α-Amylase-Inaktivator in Lösung, während Verunreinigungen wie z.B. Fremdeiweiß Niederschläge bilden können, die gegebenenfalls nach Zusatz von Fällungsmittel durch Filtration bzw. Zentrifugation entfernt werden können.

Zur klaren, sauren Tendamistat-Lösung gibt man die Lösung eines für die Kristallisation von Eiweißstoffen üblichen Salzes. Geeignete Anionen sind beispielsweise Chlorid, Bromid, Sulfat, Phosphat, Citrat, besonders geeignet ist das Chloridion. Als Chloride können zahlreiche anorganische oder organische Salze verwendet werden, wie z.B. Natriumchlorid, Kaliumchlorid, Ammoniumchlorid, Dimethylaminhydrochlorid oder ähnliche. Die Art des Kations im Salz ist von sekundärer Bedeutung. Die saure Kristallisation erfolgt in dem angegebenen pH-Bereich mit 0,1 bis 4 molarer Chloridzudosierung, vorzugsweise aber mit 0,2 bis 0,5 mol/l.

Zur Isolierung des Wirkstoffs werden die Ansätze nach 0,1 bis 28 Tagen, vorzugsweise nach 4 Tagen, gegebenenfalls nach Zusatz von Impfkristallen, zentrifugiert oder filtriert. Man erhält kristallines Tendamistat als freie Säure, dessen basische Gruppen in der Hydrochloridform, gegebenenfalls Bromid- Sulfat- oder Citratform, vorliegen. Zur weiteren Reinigung kann man die Kristallmasse in Wasser unter Natronlaugezugabe lösen, wobei der resultierende pH-Wert zwischen 5,5 und 8 liegt. Diese Lösung kann dann durch Dialyse oder durch Ultrafiltration entsalzt und anschließend nach an sich bekannten Verfahren getrocknet werden. Die Ausbeute beträgt je nach Reinheit bis über 90 % (bezogen auf den Gehalt im Kulturfiltrat).

Die Kristalle der sauren Form und der isoelektrischen Form sind nicht gleich: so kann zum Beispiel die Kristallisation neuer Ansätze nicht durch Animpfen mit der jeweils anderen Kristallart beschleunigt werden: also die isoelektrische Kristallisation nicht mit kristalliner freier Tendamistat-

0121788

- 6 -

Säure oder die Kristallisation im Sauren mit z.B. NaCl
nicht mit der isoelektrischen Tendamistatform. Demgegenüber führt das arteigene Animpfen der jeweiligen Kristallisation zur raschen Kristallbildung. Impfkristalle gewinnt
man nach den erfindungsgemäßen Verfahren, wobei als Ausgangsmaterial nach Patentschrift 27 01 890 bzw. Offenlegungsschrift
30 38 130 hergestelltes HOE 467 in Form von wäßriger Lösung
statt des Kulturfiltrates eingesetzt wird.
Durch gegebenenfalls mehrfache Umkristallisation erreicht
man Tendamistatpräparationen von großer Reinheit, vergleichbar mit Substanzen hergestellt nach einem herkömmlichen Isolier-Verfahren. Da jedoch sowohl der Material-
Einsatz als auch die erforderliche Energie vergleichsweise
gering ist bei den Kristallisationsverfahren, ist ihre Anwendung ein wertvoller Beitrag zur Gewinnung des potenten
Wirkstoffes Tendamistat.

Es war überraschend, daß Tendamistat bei den erfindungsgemäßen Verfahren kristallin und in hohen Ausbeuten anfällt, da der $\alpha$-Amylase-Inaktivator zu den Eiweißkörpern
zählt und diese hochmolekularen Verbindungen nur wenig zur
Kristallisation neigen.

Das nach den erfindungsgemäßen Verfahren erhaltene Tendamistat besitzt eine in der deutschen Offenlegungsschrift
30 38 130 beschriebene $\alpha$-Amylase hemmende Aktivität und
eignet sich daher z.B. ebenfalls für die Regulierung des
Blutzuckerspiegels.

Die nachfolgenden Beispiele erläuertn die Erfindung.

Beispiel 1

250 l der nach den Angaben der deutschen Offenlegungsschrift
30 38 130 gewonnenen Kulturlösung, wobei die Fermentationszeit auf
45 bis 60 Stunden herabgesetzt wurde, werden mit Hilfe einer Zentrifuge
(3000 G) von den ungelösten Bestandteilen befreit. Hierbei entstehen 200 l hellbraunes Kulturfiltrat enthaltend 0,5 g Tendamistat pro Liter, die mit einer Ultra-filtrationsanlage auf 20 l konzentriert und entsalzt werden.

Beispiel 2

Das Konzentrat des Beispiels 1 von 20 l wird zunächst mit 5 l Ethanol versetzt und dann mit 400 ml 10%iger Salzsäure auf pH 2,3 gestellt. Der entstandene Niederschlag von hochmolekularem Fremdeiweiß wird abfiltriert. Die klare flüssige Phase wird dann durch Ultrafiltration auf 8 l konzentriert und gewaschen, wobei der Alkoholanteil auf weniger als 1 % gesenkt wird.

Zu dieser Lösung werden 107 g Kochsalz gegeben. Anschließend wird die Lösung auf +1$^\circ$C abgekühlt und mit einigen Impfkristallen versetzt. Nach Stehenlassen über Nacht werden 3 mal täglich je 10 g Kochsalz dem Ansatz hinzugefügt bis 90 % der Aktivität sich im Kristallfiltrat befindet. Dies ist nach 3 - 4 Tagen der Fall. Nach erfolgter Kristallisation wird in der Zentrifuge bei 6000 G abzentrifugiert, die feste Wirksubstanz abgetrennt, in max. 2 l Wasser unter Natronlaugenzugabe gelöst, mit Hilfe der Ultrafiltration entsalzt und gefriergetrocknet. Die Ausbeute beträgt 72 g Tendamistat (entsprechend 72 % bezogen auf 200 l Kultur-filtrat), enthaltend mindestens 90 % HOE 467 A.

Beispiel 3

Zu 20 l des nach Beispiel 1 gewonnenen Konzentrates werden Eisessig bis zum Erreichen des pH-Wertes von 5,2 sowie 2 l Isopropanol gegeben. Der hierbei entstehende Niederschlag der Fremdeiweißverunreinigung wird abfiltriert, das klare Filtrat durch Ultrafiltration auf 10 l konzentriert, auf 6$^\circ$C gekühlt und dann mit Eisessig auf pH 4,8 gestellt. Die anfängliche klare isopropanolische Lösung kristallisiert spontan, anderenfalls wird mit einigen Impfkristallen versetzt. Nach 24 Stunden hat sich ein Kristallbrei gebildet, dessen pH-Wert auf 4,6 erniedrigt wird. Nach weiteren 48 Stunden kann abzentrifugiert und mit Wasser gewaschen werden. Die helle Kristallmasse wird dann, ohne gelöst zu werden gefriergetrocknet. Die Ausbeute beträgt 61 g (entsprechend 61 % bezogen auf 200 l Kulturfiltrat), enthaltend mindestens 90 % HOE 467 A.

0121788

Patentansprüche:

1. Verfahren zur Herstellung von Tendamistat, dadurch gekennzeichnet, daß man das Kulturfiltrat von Kulturlösungen Tendamistat-produzierender Mikroorganismen konzentriert und entsalzt und anschließend entweder

a) mit 0 bis 30 Gew.% eines geeigneten, mit Wasser mischbaren organischen Lösungsmittels versetzt, den pH-Wert dieser Lösung durch Zugabe einer in der Peptidchemie gebräuchlichen Säure auf 3,2 bis 5,5 erniedrigt oder auf 2 bis 3 erniedrigt und mit einer Base auf 3,2 bis 5,5 erhöht und das in der Lösung vorhandene Tendamistat, gegebenenfalls nach Zusatz von Impfkristallen, durch Stehenlassen zur Kristallisation bringt und abtrennt oder

b) mit einer in der Peptidchemie gebräuchlichen Säure auf einen pH-Wert zwischen 1,0 bis 3,0 einstellt, gegebenenfalls unslösliche Verunreinigungen abtrennt, die erhaltene Lösung mit einem für die Kristallisation von Eiweißstoffen üblichen Salz versetzt und das in der Lösung vorhandene Tendamistat, gegebenenfalls nach Zusatz von Impfkristallen, durch Stehenlassen zur Kristallisation bringt und abtrennt.

2. Verfahren gemäß Anspruch 1, dadurch gekennzeichnet, daß man das Kulturfiltrat der Kulturlösung von Streptomyces tendae ATCC 31 210 oder Streptomyces tendae DSM 1912 verwendet,

3. Verfahren gemäß Anspruch 1, dadurch gekennzeichnet, daß man als Säure Schwefelsäure, Salzsäure oder Essigsäure verwendet.

4. Verfahren gemäß Anspruch 1, dadurch gekennzeichnet, daß man bei Verfahren a) 5 bis 15 % Isopropanol, Ethanol oder Aceton als Lösungsmittel verwendet.

5. Verfahren gemäß Anspruch 1, dadurch gekennzeichnet, daß man bei Verfahren a) einen pH-Wert zwischen 4,2 und 5,2 einstellt.

6. Verfahren gemäß Anspruch 1, dadurch gekennzeichnet, daß man bei Verfahren b) ein Salz, dessen Anion Chlorid, Bromid, Sulfat, Phosphat oder Citrat ist, verwendet.

7. Verfahren gemäß Anspruch 1, dadurch gekennzeichnet, daß man als Salz ein Chlorid verwendet.

8. Kristallines Tendamistat erhalten nach Verfahren gemäß Anspruch 1 oder 2.

9. Kristallines Tendamistat erhalten nach Verfahren gemäß Anspruch 1 oder 2 bestehend zum überwiegenden Teil aus HOE 467 A.